# EUROPÄISCHE PATENTSCHRIFT

(11) **EP 3 456 369 B1**
(45) Veröffentlichungstag und Bekanntmachung des Hinweises auf die Patenterteilung: **27.07.2022**
(21) Anmeldenummer: 18193775.6
(22) Anmeldetag: 11.09.2018
(51) Int. Cl.: A61M 5/14, A61M 5/168

(54) **RAUMOPTIMIERTES INFUSIONSSYSTEM MIT GROSSER BEDIENER-SCHNITTSTELLE**
SPACE-OPTIMISED INFUSION SYSTEM WITH LARGE OPERATOR INTERFACE
SYSTÈME DE PERFUSION À ENCOMBREMENT OPTIMISÉ AVEC GRANDE INTERFACE OPÉRATEUR

(30) Priorität: 19.09.2017 DE 102017121677
(43) Veröffentlichungstag der Anmeldung: 20.03.2019
(73) Patentinhaber: B. Braun Melsungen AG, 34212 Melsungen (DE)
(72) Erfinder: FUCHS, Jürgen, 34308 Bad Emstal (DE); HARTUNG, Jürgen, 34298 Helsa (DE); STEGER, Jürgen, 34327 Körle (DE)
(74) Vertreter: Winter, Brandl - Partnerschaft mbB

(56) Entgegenhaltungen:
- EP-A1- 2 623 141
- EP-A2- 0 985 421
- US-A- 5 935 099

## Beschreibung

### Beschreibung

Die vorliegende Erfindung betrifft ein Infusionssystem zum Applizieren von zu infundierenden Flüssigkeiten oder Fluiden an einen Patienten mit einer Mehrzahl von insbesondere im Verbund betreibbaren Infusionspumpen und einer Bediener-Schnittstelle, auch als Interface bezeichnet, zur Eingabe von Bedienungsanweisungen durch eine Bedienperson und/oder Ausgabe oder Anzeige von Betriebsparametern an eine Bedienperson.

### Hintergrund der Erfindung

In der Infusionstechnik sind Infusionspumpen allgemein bekannt. Sie fördern in der Regel mittels eines Überleitsystems Flüssigkeit aus einem Flüssigkeitsreservoir zu einem Patienten, dem diese Flüssigkeit beispielsweise venös appliziert wird. Insbesondere im Rahmen der Intensivmedizin ist es üblich, dass mehrere Infusionspumpen im Verbund miteinander betrieben werden. Ein solcher Verbund mit einer Mehrzahl an Infusionspumpen wird mit Bezug auf die vorliegende Erfindung als Infusionssystem bezeichnet.

### Stand der Technik

Das US-Patent 5,935,099 offenbart ein Medikamentenpumpensystem und ein Verfahren dazu. Genauer wird eine Patientenpumpe und eine Pflegepumpe offenbart die miteinander in Steuerungsverbindung stehen. Die Bedienerschnittstelle der Pflegepumpe kann dabei die Patientenpumpe bedienen.

Die europäische Anmeldung 0 985 421 A1 offenbart ein medizinisches Infusionspumpensystem, bei dem mehrere Infusionspumpen ohne Bedienerschnittstelle von einer separaten Bedienerschnittstell bedient werden können.

Jede einzelne Infusionspumpe erfordert gemäß dem allgemein bekannten Stand der Technik eine Schnittstelle für eine Bedienperson oder einen Nutzer. Über eine solche Schnittstelle, die im Rahmen der Erfindung als Bediener-Schnittstelle, Interface oder Human Interface bezeichnet werden kann, werden Daten und/oder Anweisungen in eine Steuerungseinheit der Pumpe eingegeben. Außerdem werden der Bedienperson Informationen, wie zum Beispiel aktuelle Betriebsparameter der Infusionspumpe, Fehlermeldungen, etc. angezeigt. Bei bekannten Infusionssystemen, in denen mehrere Infusionspumpen im Verbund betrieben werden, ist es derzeit üblich, dass jede einzelne Infusionspumpe des Systems ihre eigene Bediener-Schnittstelle aufweist. Um eine nutzerfreundliche und ergonomische Bedienung zu ermöglichen, ist diese jeweils grundsätzlich in ausreichender Größe und an gut zugänglicher und einsehbarer Stelle jeder Infusionspumpe vorzusehen oder auszubilden. Bei bekannten Systemen ist von Nachteil, dass der große Flächen- oder Raumbedarf ergonomischer Bediener-Schnittstellen jeder einzelnen Infusionspumpe zu einem besonders großen Bauraum im Verbund (also des Infusionssystems) führt. Außerdem werden dadurch die Möglichkeiten zur räumlichen Anordnung der Pumpen im System beschränkt. Eine Ausbildung der Bediener-Schnittstellen mit kleineren Abmessungen (wie im Stand der Technik) stellt einen nachteiligen Kompromiss dar, da derart nur eine geringere Nutzerfreundlichkeit erzielt wird (schlechtere Erkennbarkeit von Anzeigen, schlechtere Bedienbarkeit von Eingabeelementen). Andere Ansätze, beispielsweise Anzeige- und Bedienelemente in einer Pumpenklappe zu integrieren, führen zu hohen Kosten und sind teils aufwändig.

### Kurzbeschreibung der Erfindung

Ausgehend davon liegt der vorliegenden Erfindung die Aufgabe zugrunde, ein Infusionssystem mit einer Mehrzahl von miteinander im Verbund angeordneten und betriebenen Infusionspumpen zu schaffen und eine besonders große und nutzerfreundliche Bediener-Schnittstelle (Human Interface) vorzusehen, die ermöglicht, alle im Verbund angeordneten Infusionspumpen des Infusionssystems einfach und nutzerfreundlich zu bedienen, ohne die vorstehend erläuterten Kompromisse des Standes der Technik bezüglich Größe, Zugänglichkeit, Bedienbarkeit und Bauraum eingehen zu müssen.

Diese Aufgabe wird durch ein Infusionssystem mit den Merkmalen des Patentanspruchs 1 gelöst.

Insbesondere wird die vorstehende Aufgabe nach der Erfindung gelöst durch ein Infusionssystem zum Applizieren von zu infundierenden Flüssigkeiten oder Fluiden an einen Patienten, aufweisend eine Mehrzahl von insbesondere im Verbund betreibbaren und zusammengeschlossenen Infusionspumpen, die jeweils zum Applizieren einer zu infundierenden Flüssigkeit vorgesehen und eingerichtet sein können, mit jeweils wenigstens einer Infusionspumpen-eigenen Bediener-Schnittstelle oder ein Interface, insbesondere mehrere Bediener-Schnittstellen oder mehrere Interfaces, zur Eingabe von die jeweilige Infusionspumpe betreffenden Bedienungsanweisungen durch eine Bedienperson und/oder Ausgabe oder Anzeige von die jeweilige Infusionspumpe betreffenden Betriebsparametern an eine Bedienperson, wobei jede der Mehrzahl von zu einem Verbund zusammengeschlossenen Infusionspumpen jeweils von der Bediener-Schnittstelle nur einer einzigen zur Bedienung verfügbaren Infusionspumpe (13) des Verbunds bedienbar ist.

Es liegt insbesondere im Rahmen der Erfindung, wenn mehrere Infusionspumpen oder jede Infusionspumpe des Verbunds jeweils eine (eigene) Bediener-Schnittstelle aufweist oder mit einer solchen ausgebildet ist. Dabei ist ein besonderer Vorteil, dass sich die Pumpen untereinander ihre eigene Bediener-Schnittstelle ausleihen können, d.h. über die Bediener-Schnittstelle einer Infusionspumpe auf weitere Infusionspumpen, insbesondere auf jede Infusionspumpe zugegriffen werden kann und derart das gesamte Infusionssystem über die Bediener-Schnittstelle einer seiner Infusionspumpen bedient werden kann. Insbesondere kann jede Bediener-Schnittstelle autark erhalten bleiben und keine zentrale Leitstelle vorgesehen sein. Dadurch wird keine zusätzliche oder separate Bediener-Schnittstelle benötigt und die redundant in den Pumpen vorhandenen Bediener-Schnittstellen können im Verbund verdeckt angeordnet sein. Außerdem können alle Pumpen / Bediener-Schnittstellen gleichberechtigt sein und Eingaben können grundsätzlich an jeder beliebigen Pumpe erfolgen. Ein weiterer Vorteil der Erfindung ist, dass sobald eine Pumpe aus dem Verbund entnommen wird, diese sofort über die nun erreichbare Bediener-Schnittstelle bedienbar ist.

Kommunikationstechnisch gekoppelt im Sinne der Erfindung bedeutet, dass eine entsprechende Infusionspumpe eine Kommunikation im weitesten Sinne, insbesondere einen Austausch von Daten und Anweisungen, mit der Bediener-Schnittstelle führt oder aufnehmen kann, insbesondere jederzeit auf eine Anweisung einer Bedienperson hin. Der Begriff "kommunikationstechnisch gekoppelt" kann daher auch eine Bedeutung im Sinne von koppelbar umfassen. Vorzugsweise verfügt jede der Infusionspumpen über eine Steuerungseinheit. Auch die Bediener-Schnittstelle verfügt über eine Steuerungseinheit, so dass eine Kommunikation zwischen den jeweiligen Steuerungseinheiten erfolgen kann. Grundsätzlich kann sie in beliebiger Weise erfolgen, zum Beispiel draht- oder kabelgebunden. Vorzugsweise erfolgt die Kommunikation zwischen den Infusionspumpen und der Bediener-Schnittstelle jedoch drahtlos oder kabellos, beispielsweise über WLAN, Bluetooth, NFC oder Infrarot. Die Erfindung kann außerdem umfassen, dass die Infusionspumpen untereinander kommunizieren.

Durch die Erfindung kann eine Mehrzahl von Infusionspumpen über eine einzige Bediener-Schnittstelle betrieben werden. Dadurch wird der Vorteil geschaffen, dass die einzelnen Infusionspumpen unabhängig von durch die Bediener-Schnittstelle zu erfüllenden Anforderungen, wie zum Beispiel einer bestimmten Größe oder Anordnung der Bediener-Schnittstelle, ausgebildet und/oder im Verbund angeordnet sein können. Die Infusionspumpen können im Verbund insbesondere bauraumoptimiert ausgebildet und/oder angeordnet werden. Es liegt im Rahmen der Erfindung, dass die Bediener-Schnittstelle unabhängig oder separat von den Infusionspumpen ausgebildet und/oder angeordnet sein kann. Außerdem kann nach der Erfindung eine (einzige) Infusionspumpe der Mehrzahl an Infusionspumpen mit der Bediener-Schnittstelle versehen sein oder diese aufweisen, während die übrigen Infusionspumpen eine Bediener-Schnittstelle nicht aufweisen und kommunikationstechnisch mit der Bediener-Schnittstelle der einen (einzigen) Infusionspumpe gekoppelt sind. Ein besonders flexibles Infusionssystem ergibt sich, wenn jede der Infusionspumpen zwar eine eigene Bediener-Schnittstelle aufweist, jedoch nur die Bediener-Schnittstelle einer einzigen "ersten" Infusionspumpe kommunikationstechnisch mit allen "übrigen" Infusionspumpen verbunden ist, derart dass eine Kommunikation zwischen einer Bedienperson und dem Infusionssystem und dessen Infusionspumpen ausschließlich über die Bediener-Schnittstelle der "ersten" Infusionspumpe ablaufen kann. Ein Vorteil ist, dass die übrigen Infusionspumpen unabhängig von ihren jeweiligen eigenen Bediener-Schnittstellen angeordnet werden können, zum Beispiel derart, dass diese verdeckt sind. Durch die Erfindung können daher mehrere Infusionspumpen im Verbund als Infusionssystem in besonders flexibler und raumsparender Weise angeordnet und betrieben werden. Insbesondere können die Infusionspumpen stapelartig aufeinander oder aneinander angeordnet sein, vor allem derart, dass die Bediener-Schnittstelle der oberen bzw. vorderen (ersten) Infusionspumpe sowie gegebenenfalls vorhandene Pumpenklappen und weitere Bedieneinheiten aller Infusionspumpen für eine Bedienperson einfach zugänglich sind. Insgesamt kann man sagen, dass durch die Erfindung ein miniaturisiertes Infusionssystem mit besonders großem Human Interface ermöglicht ist.

In anderen Worten ausgedrückt sieht der Stand der Technik bei einer gattungsgemäßen Infusionspumpe die Gehäuse-Schmalseite als Anzeige- /Bedienungsfläche vor, an/in welches die Bediener-Schnittstelle (Tastatur, Bildschirm, Touchscreen, etc. ) angeordnet sind, derart, dass auch bei einem Infusionspumpenstapel die Bediener-Schnittstelle jeder Infusionspumpe des Stapels von außen/von Vorn zugänglich ist, mit dem vorstehend genannten Nachteil, dass jede Bediener-Schnittstelle vergleichsweise klein baut (auch als "Mäusekino" bezeichnet).

Die vorliegende Erfindung sieht hingegen vor, nicht die (vordere) Schmalseite sondern die seitliche Breitseite des Infusionspumpengehäuses als Anzeige- /Bedienungsfläche zu verwenden und damit die an/in der seitlichen Breitseite ausgebildeten/angeordneten Bediener-Schnittstelle entsprechend groß und damit gut und übersichtlich bedienbar zu gestalten. Das dadurch resultierende Problem, dass im Fall eines Infusionspumpen-Stapels mit wenigstens zwei oder mehreren, an den jeweiligen seitlichen Breitseiten aneinander liegenden Infusionspumpen nur eine (oder maximal zwei) Bediener-Schnittstelle von außen zugänglich/sichtbar ist, nämlich die der einen äußersten/obersten/vordersten Infusionspumpe wird erfindungsgemäß dadurch gelöst, indem die Infusionspumpen des Infusionspumpen-Stapels steuerungstechnisch so gekoppelt werden, dass über die einzige noch von außen/oben/vorn sichtbare und zugängliche Bediener-Schnittstelle alle Infusionspumpen des Infusionspumpen-Stapels (manuell) manipuliert/eingestellt/betätigt werden können.

Beispielsweise kann dies automatisch dadurch erfolgen, indem bereits bei dem Zusammenstellen des individuellen Infusionspumpen-Stapels die jeweils benachbarten Infusionspumpen sich mechanisch, z.B. per elektrischem Steckkontakt oder kabellos z.B. per Funk steuerungstechnisch koppeln und so nur die jeweils von außen sichtbare und betätigbare (alleinige) Bediener-Schnittstelle als die zentrale Bediener-Schnittstelle für alle gekoppelten Infusionspumpen festlegen. Dies kann aber auch manuell durch entsprechendes Programmieren der einzelnen Bediener-Schnittstellen erfolgen, indem alle nicht sichtbaren/betätigbaren (weil überdeckten) Bediener-Schnittstellen quasi zu jeweils einem Empfangssatelliten programmiert/eingestellt werden und die alleinig sichtbare/betätigbare Bediener-Schnittstelle zu der zentralen Bedienungs-Leitstelle ausgewählt/programmiert wird. Insoweit ist umfasst die Erfindung grundsätzlich, jede Infusionspumpe mit Bediener-Schnittstellen zu versehen, allerdings außerdem eine Vielzahl von Infusionspumpen ohne Bediener-Schnittstellen auszubilden, die dann im Verbund/Infusionspumpen-Stapel von der einzig sichtbaren Bediener-Schnittstelle der äußersten Infusionspumpe aus betätigbar sind.

Vorteilhafte Ausführungsformen der Erfindung sind in den Unteransprüchen beansprucht und werden nachfolgend näher erläutert.

Eine Ausführungsform der Erfindung ist dadurch gekennzeichnet, dass zumindest eine der Infusionspumpen, insbesondere jede der Mehrzahl von Infusionspumpen, ein Gehäuse aufweist. Dieses kann jeweils im Wesentlichen quaderförmig ausgebildet sein. Es kann insbesondere eine Länge L, eine Breite B und eine Höhe H aufweisen. Für diese Abmessungen gilt vorzugsweise die folgende Beziehung: L > H ≥ B. Derart kann das Gehäuse in Form eines langgestreckten Quaders ausgebildet sein. Eine, mehrere oder alle Infusionspumpen können insbesondere derart ausgebildet und angeordnet sein, dass eine Vorderseite des Gehäuses die Länge L und die Höhe H aufweist, eine Oberseite des Gehäuses die Länge L und die Breite B aufweist und eine Lateralseite die Höhe H und die Breite B aufweist. Anders ausgedrückt stellt die Vorderseite des Gehäuses die größte Fläche dar. Die Bediener-Schnittstelle kann in das Gehäuse der jeweiligen Infusionspumpe eingebaut oder integriert sein. Insbesondere kann jeweils eine Bediener-Schnittstelle in das jeweilige Gehäuse jeder der Infusionspumpen eingebaut sein. Infolge der vorstehend beschriebenen im Wesentlichen quaderförmigen Gestalt des Gehäuses ist es von besonderem Vorteil, wenn die Bediener-Schnittstelle in der Vorderseite des Gehäuses angeordnet ist, da dies die größte Seite des Gehäuses und damit der Infusionspumpe ist und die Bediener-Schnittstelle in nutzerfreundlicher Weise groß und damit gut erkennbar und bedienbar ausgebildet werden kann.

Nach einer Ausführungsform kann das Gehäuse eine Pumpenklappe aufweisen. Diese kann insbesondere in einer Wand / Seite des Gehäuses angeordnet oder ausgebildet sein, die an die Wand / Seite des Gehäuses angrenzt, in der die Bediener-Schnittstelle eingebaut ist. Die Pumpenklappe kann insbesondere in eine Wand an der Oberseite des Gehäuses angeordnet oder ausgebildet sein. Insbesondere kann eine ganze Wand des Gehäuses als Pumpenklappe ausgebildet sein. Die Pumpenklappe kann einem Nutzer in einfacher Weise einen Zugang zu in dem Gehäuse befindlichen Bestandteilen der Infusionspumpe ermöglichen.

Nach einer Ausführungsform weist zumindest eine der Infusionspumpen ein insbesondere als Einmalartikel ausgebildetes Überleitsystem sowie eine mit dem Überleitsystem zusammenwirkende, insbesondere für einen mehrfachen Gebrauch ausgebildete, Verdrängereinheit zur Verdrängung von in dem Überleitsystem befindlichen zu infundierenden Fluid auf. Das Überleitsystem kann über die Pumpenklappe des Gehäuses zugänglich sein und derart ein- und ausgebaut werden. Es liegt im Rahmen der Erfindung, wenn mehrere oder sämtliche der Infusionspumpen des Infusionssystems derartig ausgebildet sind. Bei dem Überleitsystem kann es sich insbesondere um eine Spritze für eine in Form eines Spritzenvortriebs ausgebildete Verdrängereinheit oder um einen Schlauch für eine in Form einer Peristaltik ausgebildete Verdrängereinheit handeln.

Die Bediener-Schnittstelle kann insbesondere eine Ausgabeeinheit, wie einen Bildschirm und/oder Lautsprecher oder ähnliches, und/oder eine Eingabeeinheit, wie eine Tastatur, eine Maus, einen Trackball oder ähnliches, aufweisen. Vorzugweise ist sie als Touchscreen ausgebildet.

Eine Ausführungsform der Erfindung ist dadurch gekennzeichnet, dass zumindest eine der Infusionspumpen, vorzugsweise jede der Infusionspumpen, eine Statusanzeige und/oder eine Auswählelement, zum Beispiel in Form einer Auswähltaste oder Select-Taste, aufweist. Diese bzw. dieses ist vorzugsweise angeordnet an oder in einer Wand des Gehäuses, die an die Wand des Gehäuses angrenzt, in der die Bediener-Schnittstelle eingebaut ist, also vorzugsweise an der Oberseite des Gehäuses. Durch eine bedienpersonseitige Betätigung des Auswählelements einer der mehreren Infusionspumpen ist diese zum Datenaustausch mit der Bediener-Schnittstelle auswählbar. Die Statusanzeige jeder der Infusionspumpen zeigt deren jeweiligen Koppelzustand mit der Bediener-Schnittstelle an, also ob diese mit der Bediener-Schnittstelle gekoppelt oder entkoppelt ist. Dies bringt den zusätzlichen Vorteil, dass für eine Bedienperson unmittelbar diejenige Infusionspumpe erkennbar ist und angezeigt wird, deren Daten gerade über die Bediener-Schnittstelle angezeigt werden und/oder zu der Anweisungen über die Bediener-Schnittstelle eingegeben werden. Die Statusanzeige kann zum Beispiel durch ein Leuchtmittel ausgebildet sein, insbesondere ein Lämpchen oder eine Diode. Sie kann zum Beispiel eine Anzeige in unterschiedlichen Farben bewirken, so dass unterschiedliche Betriebszustände der entsprechenden Pumpe durch unterschiedliche Farben der Statusanzeige vermittelt werden. Die Statusanzeige kann dauerhaft ein- bzw. ausgeschaltet sein sowie ggf. intermittierend blinken, zum Beispiel um auf einen fehlerhaften Betriebszustand hinzuweisen.

Die Statusanzeige, das Auswählelement und die steuerungstechnische Auslegung der Infusionspumpen sind vorzugsweise derart beschaffen, dass Eingaben an der an einer beliebigen Infusionspumpe des Infusionssystems befindlichen Bediener-Schnittstelle zu derjenigen Infusionspumpe kommuniziert werden, deren Auswählelement betätigt wurde. Aus Sicherheitsgründen kann im Rahmen der Erfindung vorgesehen sein, dass nach einer erfolgten Eingabe diese zwingend an derjenigen Pumpe quittiert werden muss, deren Daten gerade in der Bediener-Schnittstelle angezeigt werden und die dies durch ihre Statusanzeige signalisiert. Hierdurch ist möglich, die Infusionspumpen des Systems hintereinander oder übereinander zu stapeln und dabei alle Bediener-Schnittstellen (bis auf eine, nämlich die der ersten, vorderen oder oberen Infusionspumpe) zu verdecken. Über die erste, vordere oder obere Pumpe ist es so möglich, alle Pumpen im Verbund zu bedienen. Es ist im Rahmen der Erfindung, wenn die Anzeige der Pumpe, an der die Select Taste betätigt wurde, an die Bediener-Schnittstellen aller Pumpen im Verbund kommuniziert und dort angezeigt wird. Diese gleichzeitige und identische Ansteuerung der Bediener-Schnittstellen erhöht in vorteilhafter Weise die Flexibilität des Infusionssystems.

Durch die Erfindung können insbesondere die folgenden Vorteile erzielt werden:
- Platzeinsparung gegenüber herkömmlichen Infusionssystemen an einem Intensiv-Arbeitsplatz. Bei zum Beispiel 20 Pumpen am Arbeitsplatz kann die erforderliche Fläche zur Bedienung der Pumpen durch die Erfindung auf 1/20 reduziert werden.
- Vergrößertes Human Interface für Infusionspumpen, dadurch erhöhte Nutzerfreundlichkeit, bessere Bedienbarkeit und geringere Fehleranfälligkeit.

Zusammenfassend kann man auch sagen, dass die Erfindung ein Infusionssystem zum Applizieren von zu infundierenden Flüssigkeiten oder Fluiden an einen Patienten betrifft. Dieses kann eine Mehrzahl von insbesondere im Verbund betreibbaren Infusionspumpen aufweisen, die jeweils zum Applizieren einer zu infundierenden Flüssigkeit vorgesehen und eingerichtet sein können. Es kann des Weiteren eine Bediener-Schnittstelle oder ein Interface aufweisen, insbesondere zumindest eine Bediener-Schnittstelle oder zumindest ein Interface, zur Eingabe von Bedienungsanweisungen durch eine Bedienperson und/oder Ausgabe oder Anzeige von Betriebsparametern an eine Bedienperson. Jede der Mehrzahl von Infusionspumpen kann jeweils kommunikationstechnisch mit der Bediener-Schnittstelle gekoppelt sein, insbesondere mit einer einzigen Bediener-Schnittstelle. Die Infusionspumpen können jeweils im Wesentlichen quaderförmig ausgebildet sein und zwei einander gegenüberliegende große Seitenflächen und dazwischen vier kleinere Seitenflächen aufweisen. Die Bediener-Schnittstelle kann an einer der beiden großen Seiten des jeweiligen der einzelnen Infusionspumpen angeordnet sein. Die Infusionspumpen können derart im Verbund gestapelt sein, dass die die Bediener-Schnittstelle aufweisende große Seite einer Infusionspumpe an der großen Seite einer anderen Infusionspumpe ohne Bediener-Schnittstelle anliegt. Auf diese Weise ist die Bediener-Schnittstelle einer einzigen "ersten" Infusionspumpe im Verbund nicht von den übrigen Infusionspumpen des Verbunds verdeckt, und alle Infusionspumpen des Verbunds sind kommunikationstechnisch zumindest mit der Bediener-Schnittstelle der "ersten" Infusionspumpe verbunden.

Weitere Merkmale und Vorteile der vorliegenden Erfindung ergeben sich aus der folgenden beispielhaften und nicht beschränkenden Beschreibung der Erfindung anhand von Figuren. Diese sind lediglich schematischer Natur und dienen nur dem Verständnis der Erfindung. Dabei zeigen:
Fig. 1 ein Infusionssystem nach dem Stand der Technik,
Fig. 2 eine Infusionspumpe eines erfindungsgemäßen Infusionssystems in einer perspektivischen Ansicht und
Fig. 3 ein erfindungsgemäßes Infusionssystem mit fünf Infusionspumpen nach Art der Figur 2 in einer perspektivischen Ansicht.

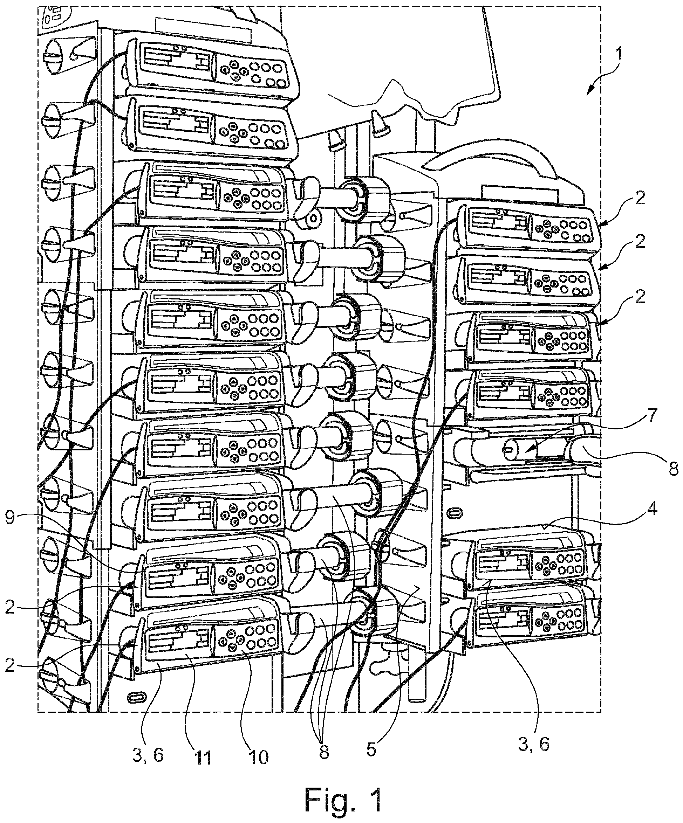

Figur 1 zeigt ein Infusionssystem 1 nach dem Stand der Technik. Dieses besteht aus mehreren einzelnen Infusionspumpen 2, die im Verbund betrieben werden. Jede der Infusionspumpen 2 weist eine Vorderseite 3, eine Oberseite 4 und eine laterale Seite 5 auf. An der Vorderseite 3 ist jeweils eine Pumpenklappe 6 ausgebildet, die einen in der Figur 1 auf der rechten Seite erkennbaren Aufnahmeraum 7 für ein austauschbares Überleitsystem 8, hier jeweils in Form von Einwegspritzen 8, abdeckt und durch Verschwenken öffnen kann. In der Pumpenklappe 6 ist jeweils eine eigene Bediener-Schnittstelle 9 für jede Pumpe 2 angeordnet, jeweils mit einem Eingabebereich 10 und einem Display 11 als Ausgabebereich.

Bei dem bekannten Infusionssystem 1 der Figur 1 ist jede Infusionspumpe 2 derart ausgebildet und angeordnet, dass die Oberseite 4 die größte Seite der Pumpe ist, während die Vorderseite 3 von geringerer Größe ist und insbesondere flach ausgebildet ist. Die einzelnen Infusionspumpen 2 des Systems 1 sind übereinander gestapelt, also jeweils Unterseite auf Oberseite, sodass die Vorderseiten 3 jeder Pumpe für eine Bedienperson sichtbar und zugänglich sind. Die Abmessungen der Bediener-Schnittstelle 9 sind durch die Größe der Vorderseite 3 der Infusionspumpe 2 beschränkt.

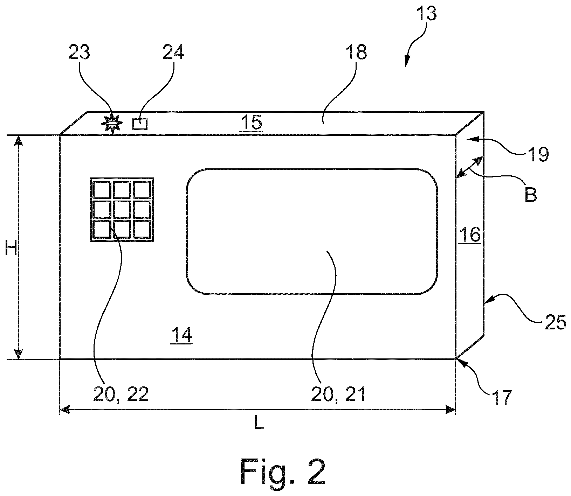

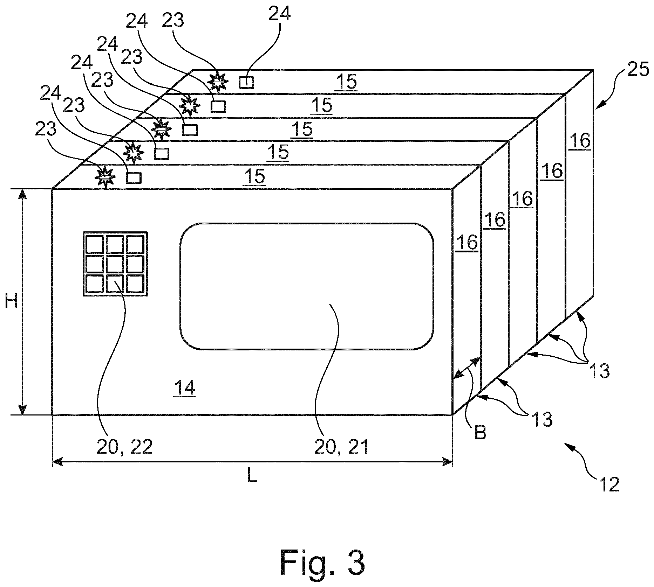

Das erfindungsgemäße Infusionssystem (12), das in Figur 3 gezeigt ist, weist ebenfalls eine Mehrzahl von Infusionspumpen 13 auf, im vorliegenden Beispiel insgesamt fünf. Eine der Infusionspumpen 13 ist einzeln in Figur 2 dargestellt. Es sei darauf hingewiesen, dass alle Infusionspumpen 13 des beispielhaften Infusionssystems 12 gleich ausgebildet sein können. Dies ist allerdings erfindungsgemäß nicht zwingend erforderlich. Die in Figur 2 gezeigte Infusionspumpe 13 weist eine Vorderseite 14 mit einer Länge L und einer Höhe H, eine Oberseite 15 mit der Länge L und einer Breite B sowie eine laterale Seite 16 mit der Höhe H und der Breite B auf. Figur 2 zeigt deutlich, dass die Abmessungen der Pumpe 13 die Beziehung L > H ≥ B erfüllen. Infolgedessen ist die Vorderseite 14 die Seite der Infusionspumpe mit der größten Fläche, gefolgt von der Oberseite 15 und schließlich der lateralen Seite 16. Die Seiten 14, 15, 16 der Infusionspumpe 13 sind durch deren Gehäuse 17 ausgebildet.

Die Oberseite 15 umfasst eine Pumpenklappe 18, die einen Aufnahmeraum 19 abdeckt bzw. freigibt, hinter der sich das Überleitsystem 8 in Form eines Einmalartikels, beispielsweise eine Spritze 8 oder ein Schlauch 8, befindet. Das Überleitsystem 8 wird mittels einer in den Figuren 2 und 3 nicht dargestellten Verdrängereinheit, zum Beispiel in Form eines Linearantriebs für eine Spritze 8 oder einer Peristaltik für einen Schlauch 8 betätigt. Entsprechend der Erfindung und abweichend vom Stand der Technik befindet sich die Pumpenklappe 18 nicht in oder an der großen Vorderseite 14, sondern an der kleineren Oberseite 15 der Infusionspumpe 13.

Durch die Erfindung ist es möglich, die große und gut einsehbare und zugängliche Vorderseite 14 gegebenenfalls sogar vollflächig zur Anordnung und Aufnahme einer Bediener-Schnittstelle 20 zu nutzen. Diese kann daher in vorteilhafter Weise groß, mit guter Zugänglichkeit, guter Bedienbarkeit und guter Erkennbarkeit ausgebildet sein. Die Bediener-Schnittstelle weist insbesondere ein großformatiges Display 21, das als Touchscreen 21 ausgebildet sein kann, und eine Tastatur 22 auf. Für den Fall, dass das Display 21 als Touchscreen 21 ausgebildet ist, kann die Tastatur 22 selbstverständlich entfallen.

An der Oberseite 15 der Infusionspumpe 13 sind in oder neben der Pumpenklappe 18 eine Statusanzeige 23 und eine Auswähltaste 24 als Auswählelement angeordnet. Im vorliegenden Ausführungsbeispiel verfügt die Statusanzeige 23 über eine Diode, so dass eine Anzeige in mehreren unterschiedlichen Farben möglich ist. Über die Statusanzeige 23 werden Informationen zur entsprechenden Pumpe 13 direkt an dieser selbst angezeigt, so dass stets eine eindeutige Zuordnung von Pumpe 13 und der jeweiligen Anzeige gegeben ist. Zum Beispiel kann eine Infusionspumpe 13 im Verbund signalisieren, dass eine Betriebsstörung vorliegt, indem die Statusanzeige 23 z.B. rot blinkt. Außerdem kann zum Beispiel durch eine gelbe blinkende Anzeige mitgeteilt werden, dass die entsprechende Pumpe 13 im Verbund durch Betätigung der AuswählTaste 24 ausgewählt wurde, sich aber noch nicht in Betrieb befindet, also es sich um die Pumpe 13 handelt, deren Daten oder Informationen gerade auf dem Display 21 der Bediener-Schnittstelle 20 zur Anzeige gebracht werden. Schließlich kann durch eine grüne Daueranzeige indiziert werden, dass sich die Pumpe 13 in störungsfreiem Betrieb befindet. Es sei darauf hingewiesen, dass alle Pumpen 13 des Infusionssystems 12 derart ausgebildet sein können, insbesondere dass alle Pumpen 13 mit einer Bediener-Schnittstelle 20 versehen sind.

Figur 3 zeigt, dass die einzelnen Infusionspumpen 13 in einem Verbund hintereinander als Infusionssystem 12 angeordnet sind. Die Anordnung der Pumpen 13 erfolgt derart, dass die Vorderseite 14 der "ersten" Infusionspumpe 13 unverdeckt ist. Die Vorderseiten der weiteren Infusionspumpen 13 liegen jeweils an der Rückseite 25 (die der Vorderseite 14 jeweils gegenüberliegende Seite der Infusionspumpe 13) an. Die Vorderseiten 13 und damit die Bediener-Schnittstellen 20 aller Infusionspumpen 13 mit Ausnahme der "ersten" (vorderen oder oberen) Infusionspumpe 13 sind daher jeweils von anderen Infusionspumpen 13 verdeckt. Unverdeckt und frei bleiben jeweils die Oberseiten 15 aller Infusionspumpen 13, so dass, wie deutlich aus Figur 3 hervorgeht, die dort befindlichen Pumpenklappen 18 sowie die Statusanzeigen 23 und die Auswähl-Elemente 24 für einen Bediener gut erkennbar und zugänglich sind. Es sei darauf hingewiesen, dass der Verbund des in Figur 3 gezeigten Infusionssystems 12 außerdem mit der Pumpenklappe 18 nach vorne und der Bediener-Schnittstelle 20 nach oben weisend betrieben werden kann. Eine Drehung des Infusionssystems 12 im Raum ist in beliebige Stellungen möglich, je nach den jeweils vorliegenden Möglichkeiten im Hinblick auf eine Zugänglichkeit.

### Bezugszeichenliste

- 1: Infusionssystem
- 2: Infusionspumpe
- 3: Vorderseite
- 4: Oberseite
- 5: laterale Seite / Lateralseite
- 6: Pumpenklappe
- 7: Aufnahmeraum
- 8: Überleitungssystem, Einwegspritze
- 9: Bediener-Schnittstelle
- 10: Eingabebereich, Tastatur
- 11: Ausgabebereich, Display
- 12: Infusionssystem
- 13: Infusionspumpe
- 14: Vorderseite
- 15: Oberseite
- 16: laterale Seite / Lateralseite
- 17: Gehäuse
- 18: Pumpenklappe
- 19: Aufnahmeraum
- 20: Bediener-Schnittstelle
- 21: Ausgabeeinheit / Display
- 22: Eingabeeinheit / Tastatur
- 23: Statusanzeige
- 24: Auswählelement, Auswähltaste
- 25: Rückseite
- L: Länge
- H: Höhe
- B: Breite

## Patentansprüche

1. Infusionssystem (12) zum Applizieren von zu infundierenden Flüssigkeiten an einen Patienten, aufweisend eine Mehrzahl von zu einem Verbund zusammengeschlossenen Infusionspumpen (13) mit jeweils wenigstens einer Infusionspumpen-eigenen Bediener-Schnittstelle (20) zur Eingabe von die jeweilige Infusionspumpe betreffenden Bedienungsanweisungen durch eine Bedienperson und/oder Ausgabe von die jeweilige Infusionspumpe betreffenden Betriebsparametern an eine Bedienperson, wobei
jede der Mehrzahl von zu einem Verbund zusammengeschlossenen Infusionspumpen (13) jeweils von der Bediener-Schnittstelle (20) nur einer einzigen zur Bedienung verfügbaren Infusionspumpe (13) des Verbunds bedienbar ist, **dadurch gekennzeichnet dass** bei wenigstens zwei aneinander liegenden Infusionspumpen (13), die einen Verbund bilden, nur eine "erste" Bediener-Schnittstelle (20) von außen zugänglich und/oder sichtbar und/oder betätigbar ist.

2. Infusionssystem (12) nach Anspruch 1, **dadurch gekennzeichnet, dass** die einzige zur Verfügung stehende Bediener-Schnittstelle (20) als die zentrale Bediener-Schnittstelle für alle gekoppelten Infusionspumpen (13) festlegbar ist.

3. Infusionssystem (12) nach Anspruch 1 oder 2, **dadurch gekennzeichnet, dass** eine Kommunikation zwischen der Bedienperson und dem Infusionssystem (12) und dessen Infusionspumpen (13) ausschließlich über die Bediener-Schnittstelle (20) der einzigen zur Bedienung verfügbaren Infusionspumpe (13) des Verbundes erfolgt.

4. Infusionssystem (12) nach einem der vorstehenden Ansprüche, **dadurch gekennzeichnet, dass** die Infusionspumpen (13) stapelartig aufeinander oder aneinander zu einem Verbund angeordnet sind, derart, dass nur die Bediener-Schnittstelle (20) einer "ersten" Infusionspumpe (13) zugänglich ist, wohingegen vorzugsweise alle anderen Bediener-Schnittstellen (20) der dem Verbund zugehörigen Infusionspumpen (13) jeweils gegenseitig verdeckt sind/ werden.

5. Infusionssystem (12) nach einem der vorstehenden Ansprüche, **dadurch gekennzeichnet, dass** die jeweils benachbarten Infusionspumpen (13) mechanisch oder kabellos steuerungstechnisch koppelbar sind.

6. Infusionssystem (12) nach einem der vorstehenden Ansprüche, **dadurch gekennzeichnet, dass** zumindest eine der Infusionspumpen (13), insbesondere jede der Mehrzahl von Infusionspumpen (13), ein Gehäuse (17) aufweist, wobei die Bediener-Schnittstelle (20) jeder Infusionspumpe (13) des Verbundes insbesondere ein Touchscreen oder eine Tastatur mit einem Bildschirm ist.

7. Infusionssystem (12) nach Anspruch 6, **dadurch gekennzeichnet, dass** das Gehäuse (17) im Wesentlichen quaderförmig mit einer Länge L, einer Breite B und einer Höhe H ausgebildet ist, wobei gilt L > H ≥ B, wobei eine Vorderseite (14) des Gehäuses (17) die Länge L und die Höhe H aufweist, eine Oberseite (15) des Gehäuses (17) die Länge L und die Breite B aufweist und eine Lateralseite (16) des Gehäuses (17) die Höhe H und die Breite B aufweist.

8. Infusionssystem (12) nach Anspruch 6 oder 7, **dadurch gekennzeichnet, dass** die Bediener-Schnittstelle (20) in das Gehäuse (17) zumindest einer der Infusionspumpen (13) eingebaut ist, insbesondere dass jeweils eine Bediener-Schnittstelle (20) in das Gehäuse (17) jeder der Infusionspumpen (13) eingebaut ist.

9. Infusionssystem (12) nach einem der Ansprüche 7 oder 8, **dadurch gekennzeichnet, dass** die Bediener-Schnittstelle (20) in der Vorderseite (14) des Gehäuses (17) angeordnet ist.

10. Infusionssystem (12) nach einem der Ansprüche 7 bis 9, **dadurch gekennzeichnet, dass** das Gehäuse (17) eine Pumpenklappe (18) aufweist.

11. Infusionssystem (12) nach einem der Ansprüche 7 bis 10, **dadurch gekennzeichnet, dass** die Infusionspumpen (13) stapelartig aufeinander oder aneinander angeordnet sind, derart, dass die jeweilige Vorderseiten (14), mit Ausnahme der ersten Infusionspumpe (13), jeweils einer benachbarten Infusionspumpe (13) zugewandt sind und die Pumpenklappen (18) aller Infusionspumpen (13) für eine Bedienperson zugänglich sind.

12. Infusionssystem (12) nach Anspruch 10 oder 11, **dadurch gekennzeichnet, dass** die Pumpenklappe (18) in einer Wand oder Seite (15) des Gehäuses (17) angeordnet ist, die an eine Wand oder Seite (14) des Gehäuses (17) angrenzt, in der die Bediener-Schnittstelle (20) eingebaut ist, insbesondere in eine Wand an der Oberseite (15) des Gehäuses (17).

13. Infusionssystem (12) nach einem der vorstehenden Ansprüche, **dadurch gekennzeichnet, dass** zumindest eine der Infusionspumpen (13) ein insbesondere als Einmalartikel ausgebildetes Überleitsystem (8) sowie eine mit dem Überleitsystem (8) zusammenwirkende, insbesondere für einen mehrfachen Gebrauch ausgebildete, Verdrängereinheit zur Verdrängung von in dem Überleitsystem (8) befindlichen zu infundierenden Fluid aufweist.

14. Infusionssystem (12) nach einem der vorstehenden Ansprüche, **dadurch gekennzeichnet, dass** zumindest eine der Infusionspumpen (13), vorzugsweise jede der Infusionspumpen (13), eine Statusanzeige (23) und/oder ein Auswählelement (24) aufweist, vorzugsweise angeordnet an oder in einer Wand (15) des Gehäuses (17), die an eine Wand (14) des Gehäuses (17) angrenzt, in der die Bediener-Schnittstelle (20) eingebaut ist.

15. Infusionssystem (12) nach Anspruch 8, **dadurch gekennzeichnet, dass** zumindest eine der Infusionspumpen (13), vorzugsweise jede der Infusionspumpen (13), durch bedienpersonseitige Betätigung des Auswählelements (24) zum Datenaustausch mit der Bediener-Schnittstelle (20) auswählbar ist und/oder dass die Statusanzeige (24) der jeweiligen Infusionspumpe (13) einen Koppelzustand dieser Infusionspumpe (13) mit der Bediener-Schnittstelle (20) anzeigt.

## Claims

1. An infusion system (12) for applying fluids to be infused to a patient, comprising a plurality of infusion pumps (13) combined into a group, each having at least one inbuilt infusion pump operator interface (20) for inputting the respective operating instructions relating to the infusion pump by an operator and/or for outputting the operating parameters relating to the respective infusion pump to an operator, wherein each of the plurality of infusion pumps (13) combined into a group can be operated respectively from the operator interface (20) of only a single infusion pump (13) available for operation, **characterized in that** in the case of at least two adjacent infusion pumps (13), which form a group, only a "first" operator interface (20) is accessible and/or visible and/or actuable from outside.

2. The infusion system (12) according to claim 1, **characterized in that** the only available operator interface (20) can be defined as the central operator interface for all coupled infusion pumps (13).

3. The infusion system (12) according to claim 1 or 2, **characterized in that** a communication between the operator and the infusion system (12) and the infusion pumps (13) thereof occurs exclusively via the operator interface (20) of the only infusion pump (13) of the group available for operation.

4. The infusion system (12) according to any of the foregoing claims, **characterized in that** the infusion pumps (13) are arranged in the form of a stack on top of one another or on one another into a group, such that only the operator interface (20) of a "first" infusion pump (13) is accessible, preferably all other operator interfaces (20) of the infusion pumps (13) belonging to the group are or will be respectively mutually covered.

5. The infusion system (12) according to any of the foregoing claims, characterized that the respective adjacent infusion pumps (13) can be coupled mechanically or wirelessly in terms of control technology.

6. The infusion system (12) according to any of the foregoing claims, **characterized in that** at least one of the infusion pumps (13), in particular each of the plurality of infusion pumps (13), comprises a housing (17), wherein the operator interface (20) of each infusion pump (13) of the group is in particular a touch screen or a keyboard with a monitor.

7. The infusion system (12) according to claim 6, **characterized in that** the housing (17) is embodied substantially cuboid with a length L, a width B and a height H, wherein L>H≥B, wherein a front side (14) of the housing (17) comprises the length L and the height H, an upper side (15) of the housing (17) comprises the length L and the width B and a lateral side (16) of the housing (17) comprises the height H and the width B.

8. The infusion system (12) according to claim 6 or 7, **characterized in that** the operator interface (20) is built into the housing (17) of at least one of the infusion pumps (13), in particular **in that** in each case an operator interface (20) is built into the housing (17) of each of the infusion pumps (13).

9. The infusion system (12) according to any of claims 7 or 8, **characterized in that** the operator interface (20) is arranged in the front side (14) of the housing (17).

10. The infusion system (12) according to any of claims 7 to 9, **characterized in that** the housing (17) comprises a pump valve (18).

11. The infusion system (12) according to any of claims 7 to 10, **characterized in that** the infusion pumps (13) are arranged in the form of a stack on top of one another such that the respective front sides (14), with the exception of the first infusion pump (13), each are facing an adjacent infusion pump (13) and the pump valves (18) of all infusion pumps (13) are accessible for an operator.

12. The infusion system (12) according to claim 10 or 11, **characterized in that** the pump valve (18) is arranged in a wall or side (15) of the housing (17), which adjoins a wall or side (14) of the housing (17) into which the operator interface (20) is built, in particular in a wall on the upper side (15) of the housing (17).

13. The infusion system (12) according to any of the foregoing claims, **characterized in that** at least one of the infusion pumps (13) comprises a transfer system (8) in particular embodied as disposable item and a displacement unit interacting with the transfer system (8) designed for multiple use for displacement of fluid in the transfer system (8) to be infused.

14. The infusion system (12) according to any of the foregoing claims, **characterized in that** at least one of the infusion pumps (13), preferably each of the infusion pumps (13) comprises a status display (23) and/or a selection element (24), preferably arranged on or in a wall (15) of the housing (17), which adjoins a wall (14) of the housing (17), in which the operator interface (20) is built.

15. The infusion system (12) according to claim 8, **characterized in that** at least one of the infusion pumps (13), preferably each of the infusion pumps (13), is selectable by operator actuation of the selection element (24) for data exchange with the operator interface (20) and/or **in that** the status display (24) of the respective infusion pump (13) displays a coupling state of this infusion pump (13) with the operator interface (20).

## Revendications

1. Système de perfusion (12) pour appliquer des liquides à perfuser à un patient, présentant une pluralité de pompes à perfusion (13) regroupées en un ensemble avec respectivement au moins une interface utilisateur (20) propre à la pompe à perfusion pour l'entrée de manuels d'utilisation concernant la pompe à perfusion respective par un utilisateur et/ou la délivrance de paramètres de fonctionnement concernant la pompe à perfusion respective à un utilisateur, dans lequel chacune de la pluralité de pompes à perfusion (13) regroupées en un ensemble peut être commandée respectivement par l'interface utilisateur (20) d'une seule pompe à perfusion (13) de l'ensemble disponible pour une utilisation, **caractérisé en ce que** lorsqu'au moins deux pompes à perfusion (13), qui forment un ensemble, sont situées l'une sur l'autre, seule une « première » interface utilisateur (20) est accessible et/ou visible et/ou peut être actionnée de l'extérieur.

2. Système de perfusion (12) selon la revendication 1, **caractérisé en ce que** la seule interface utilisateur (20) disponible peut être définie comme l'interface utilisateur centrale pour toutes les pompes à perfusion (13) accouplées.

3. Système de perfusion (12) selon la revendication 1 ou 2, **caractérisé en ce qu'**une communication entre l'utilisateur et le système de perfusion (12) et les pompes à perfusion (13) de celui-ci s'effectue exclusivement par l'intermédiaire de l'interface utilisateur (20) de la seule pompe à perfusion (13) de l'ensemble disponible pour l'utilisation.

4. Système de perfusion (12) selon l'une quelconque des revendications précédentes, **caractérisé en ce que** les pompes à perfusion (13) sont disposées en piles les unes sur les autres ou les unes contre les autres en un ensemble, de telle sorte que seule l'interface utilisateur (20) d'une « première » pompe à perfusion (13) est accessible, tandis que de préférence toutes les autres interfaces utilisateur (20) des pompes à perfusion (13) faisant partie de l'ensemble sont/deviennent respectivement mutuellement cachées.

5. Système de perfusion (12) selon l'une quelconque des revendications précédentes, **caractérisé en ce que** les pompes à perfusion (13) respectivement voisines peuvent être accouplées mécaniquement ou sans fil par technique de commande.

6. Système de perfusion (12) selon l'une quelconque des revendications précédentes, **caractérisé en ce qu'**au moins une des pompes à perfusion (13), en particulier chacune de la pluralité de pompes à perfusion (13), présente un boîtier (17), dans lequel l'interface utilisateur (20) de chaque pompe à perfusion (13) de l'ensemble est en particulier un écran tactile ou un clavier avec un écran.

7. Système de perfusion (12) selon la revendication 6, **caractérisé en ce que** le boîtier (17) est réalisé sensiblement de manière parallélépipédique avec une longueur L, une largeur B et une hauteur H, dans lequel L > H ≥ B s'applique, dans lequel une face avant (14) du boîtier (17) présente la longueur L et la hauteur H, une face supérieure (15) du boîtier (17) présente la longueur L et la largeur B et une face latérale (16) du boîtier (17) présente la hauteur H et la largeur B.

8. Système de perfusion (12) selon la revendication 6 ou 7, **caractérisé en ce que** l'interface utilisateur (20) est montée dans le boîtier (17) d'au moins une des pompes à perfusion (13), en particulier que respectivement une interface utilisateur (20) est montée dans le boîtier (17) de chacune des pompes à perfusion (13).

9. Système de perfusion (12) selon l'une quelconque des revendications 7 ou 8, **caractérisé en ce que** l'interface utilisateur (20) est disposée dans la face avant (14) du boîtier (17) .

10. Système de perfusion (12) selon l'une quelconque des revendications 7 à 9, **caractérisé en ce que** le boîtier (17) présente un clapet de pompe (18).

11. Système de perfusion (12) selon l'une quelconque des revendications 7 à 10, **caractérisé en ce que** les pompes à perfusion (13) sont disposées les unes sur les autres ou les unes contre les autres en piles, de telle sorte que les faces avant (14) respectives, à l'exception de la pompe à perfusion (13), sont respectivement tournées vers une pompe à perfusion (13) voisine et les clapets de pompe (18) de toutes les pompes à perfusion (13) sont accessibles à un utilisateur.

12. Système de perfusion (12) selon la revendication 10 ou 11, **caractérisé en ce que** le clapet de pompe (18) est disposé dans une paroi ou face (15) du boîtier (17) qui est adjacente à une paroi ou face (14) du boîtier (17) dans laquelle l'interface utilisateur (20) est montée, en particulier dans une paroi sur la face supérieure (15) du boîtier (17).

13. Système de perfusion (12) selon l'une quelconque des revendications précédentes, **caractérisé en ce qu'**au moins une des pompes à perfusion (13) présente un système de transfert (8) réalisé en particulier sous la forme d'un article jetable ainsi qu'une unité de refoulement coopérant avec le système de transfert (8), réalisée en particulier pour un usage multiple, pour le refoulement du fluide à perfuser se trouvant dans le système de transfert (8).

14. Système de perfusion (12) selon l'une quelconque des revendications précédentes, **caractérisé en ce qu'**au moins une des pompes à perfusion (13), de préférence chacune des pompes à perfusion (13), présente un indicateur de statut (23) et/ou un élément de sélection (24), de préférence disposé sur ou dans une paroi (15) du boîtier (17) qui est adjacente à une paroi (14) du boîtier (17) dans laquelle l'interface utilisateur (20) est montée.

15. Système de perfusion (12) selon la revendication 8, **caractérisé en ce qu'**au moins une des pompes à perfusion (13), de préférence chacune des pompes à perfusion (13), peut être sélectionnée par un actionnement côté utilisateur de l'élément de sélection (24) pour l'échange de données avec l'interface utilisateur (20) et/ou que l'indicateur de statut (24) de la pompe à perfusion (13) respective indique un état d'accouplement de cette pompe à perfusion (13) à l'interface utilisateur (20).
